# EUROPÄISCHE PATENTSCHRIFT

(11) **EP 1 916 990 B1**
(45) Veröffentlichungstag und Bekanntmachung des Hinweises auf die Patenterteilung: **03.11.2010**
(21) Anmeldenummer: 06762725.7
(22) Anmeldetag: 20.07.2006
(51) Int. Cl.: A61K 8/67, A61K 8/44, A61K 8/49, A61Q 17/04, A61Q 19/08

(54) **STABILE WIRKSTOFFKOMBINATIONEN AUF DER GRUNDLAGE VON FOLSÄURE**
STABLE ACTIVE-INGREDIENT COMBINATIONS BASED ON FOLIC ACID
COMBINAISONS D'AGENTS ACTIFS STABLES A BASE D'ACIDE FOLIQUE

(30) Priorität: 01.08.2005 DE 102005036093
(43) Veröffentlichungstag der Anmeldung: 07.05.2008
(73) Patentinhaber: Beiersdorf Aktiengesellschaft, 20245 Hamburg (DE)
(72) Erfinder: ECKERT, Julia, 22085 Hamburg (DE); MAX, Heiner, 22529 Hamburg (DE); RASCHKE, Thomas, 25421 Pinneberg (DE); TREU, Jens, 22844 Norderstedt (DE)
(86) Internationale Anmeldenummer: PCT/EP2006/007133
(87) Internationale Veröffentlichungsnummer: WO 2007/014644

(56) Entgegenhaltungen:
- EP-A- 1 214 927
- EP-A- 1 609 462
- DE-A1- 4 341 001
- US-A- 2 695 860
- US-B2- 6 500 472

## Beschreibung

Die vorliegende Erfindung betrifft stabile Wirkstoffkombinationen auf der Grundlage von Folsäure sowie die Verwendung bestimmter Zusatzstoffe zur Stabilisierung von Folsäure in kosmetischen Zubereitungen.

Die vorliegende Erfindung betrifft dementsprechend in bevorzugten Ausführungsformen kosmetische bzw. dermatologische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Die menschliche Haut übt als größtes Organ des Menschen zahlreiche lebenswichtige Funktionen aus. Mit durchschnittlich etwa 2 m² Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu.

Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen sowie ihre Hornschicht bei aufgetretenen Schäden in ihrem natürlichen Regenerationsvermögen zu unterstützen.

Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die vorliegende Erfindung betrifft ferner Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung kosmetischer oder dermatologischer Hautveränderungen wie z.B. der Hautalterung, insbesondere der durch oxidative Prozesse hervorgerufenen Hautalterung.

Weiterhin betrifft die vorliegende Erfindung Wirkstoffe und Zubereitungen, solche Wirkstoffe enthaltend, zur kosmetischen und dermatologischen Behandlung oder Prophylaxe erythematöser, entzündlicher, allergischer oder autoimmunreaktiver Erscheinungen, insbesondere Dermatosen.

Die vorliegende Erfindung betrifft in einer weiteren vorteilhaften Ausführungsform Wirkstoffkombinationen und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt. Während Strahlen mit einer Wellenlänge, die kleiner als 290 nm ist (der so genannte UVC-Bereich), von der Ozonschicht in der Erdatmosphäre absorbiert werden, verursachen Strahlen im Bereich zwischen 290 nm und 320 nm, dem so genannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, dass UVA-Strahlung zu einer Schädigung der elastischen und kollagenen Fasern des Bindegewebes führt, was die Haut vorzeitig altern läßt, und dass sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, dass auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Hauptsächlich werden Antioxidantien als Schutzsubstanzen gegen den Verderb der sie enthaltenden Zubereitungen verwendet. Dennoch ist bekannt, dass auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt. Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Auch die Haut, insbesondere die Altershaut, aber auch die Kopfhaut ist chemischen und physikalischen Streßfaktoren (Toxinen, UV-Licht) ausgesetzt.

Zur Behandlung der Schäden und auch vorbeugend ist es bekannt, den genannten Zubereitungen als Wirkstoffe Antioxidantien z.B. Vitamine zuzugeben. Dabei ist es ein großes Problem, Grundlage und Wirkstoffe so abzustimmen, dass die Wirkstoffe eine ausreichende Lagerstabilität erlangen, denn diese Wirkstoffe besitzen eine unterschiedliche Empfindlichkeit gegenüber oxidativen Einflüssen und Oxidationsmittel (Sauerstoff, Licht) und Wärme.

Als Lösungen dieses Problems ist schon vorgeschlagen worden, die Wirkstoffe durch die Zugabe eines oder mehrerer Stabilisatoren, z.B. anderen Antioxidantien vor schnellem Abbau zu bewahren oder sie in der Form stabiler Derivate einzusetzen. Häufig bleibt aber die erzielte Wirkung hinter der erhofften Wirkung zurück.

Aufgabe der Erfindung war es daher, hier Abhilfe zu schaffen und stabile topische Zubereitungen mit oxidationsempfindlichen Wirkstoffen zu schaffen, deren Wirkstoffgehalt über einen langen Zeitraum erhalten bleibt.

Die kosmetische und dermatologische Verwendung von Folsäure ist an sich bekannt. So beschreibt die DE 100 62 401 die Verwendung von Folsäure und/oder deren Derivaten zur Herstellung kosmetischer oder dermatologischer Zubereitungen zur Prophylaxe von Schäden an der hauteigenen DNA und/oder zur Reparatur bereits eingetretener Schäden an der hauteigenen DNA.

Folsäure weist folgende Struktur auf:

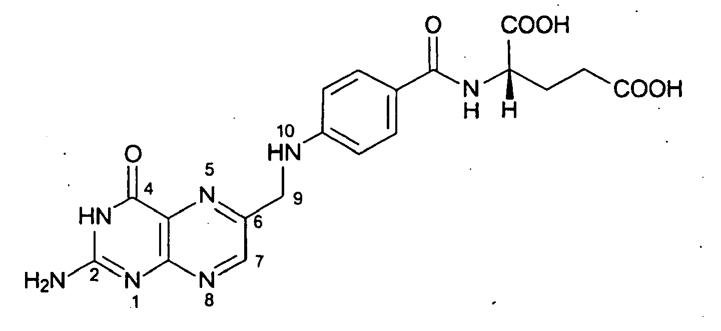

Folsäure kommt in Leber, Niere, Hefe, Pilzen, Getreide und grünen Blättern, vorwiegend als Konjugat mit Poly-□-L-glutaminsäure (Pteroylpolyglutaminsäuren) vor. Sie wurde als Wuchsstoff für verschiedene Mikroorganismen entdeckt und hat für den menschlichen Organismus Vitamincharakter. Der Bedarf des erwachsenen Menschen beträgt etwa 200 µg je Tag an bioverfügbarem Folat.

Folsäure steht im Organismus im Gleichgewicht mit 7,8-Dihydrofolsäure (H₂Folat; alte Abkürzung: FH₂) unter Beteiligung von Nicotinamid-Adenin-Dinucleotid-Phosphat und des Enzyms Dihydrofolat-Reduktase. H₂Folat wiederum entsteht in Pflanzen und einigen Mikroorganismen über mehrere Zwischenstufen aus Guanosin-5'-triphosphat (Guanosinphosphate) und setzt sich mit Hilfe der Dihydrofolat-Reduktase zu (6S)-5,6,7,8-Tetrahydrofolsäure um, der eigentlichen physiologisch wirksamen Form der Folsäure.

7,8-Dihydrofolsäure hat folgende chemische Struktur:

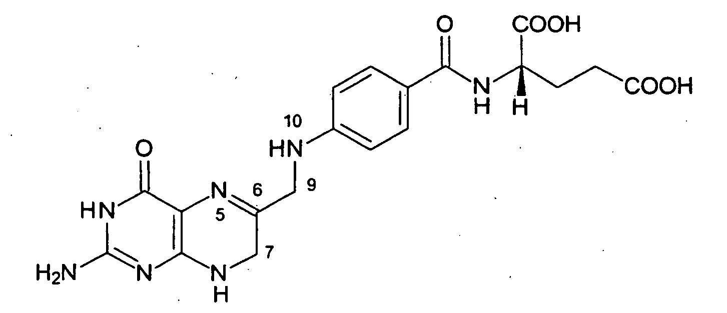

(6S)-5,6,7,8-Tetrahydrofolsäure hat folgende chemische Struktur:

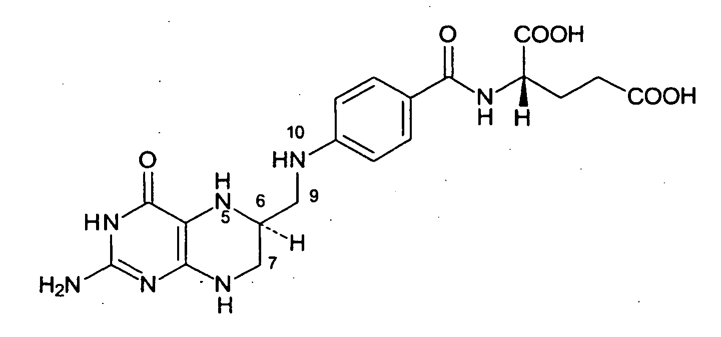

Unter dem Begriff der Derivate der Folsäure sind insbesondere die vorgenannte Dihydrofolsäure und die Tetrahydrofolsäure zu verstehen.

Nachteilig ist jedoch, dass Folsäure und ihre Derivate photolabil sind und der Stand der Technik bislang nur unzureichende Mittel zur Verfügung stellte, Folsäure und Derivate vor photoinduziertem Verfall zu schützen

Ein bekannter Lichtschutzfilter ist das 2,4-Bis-{[4-(2-ethyl-hexyloxy)-2-hydroxy]-phenyl}-6-(4-methoxyphenyl)-1,3,5-triazin, das durch die chemische Strukturformel 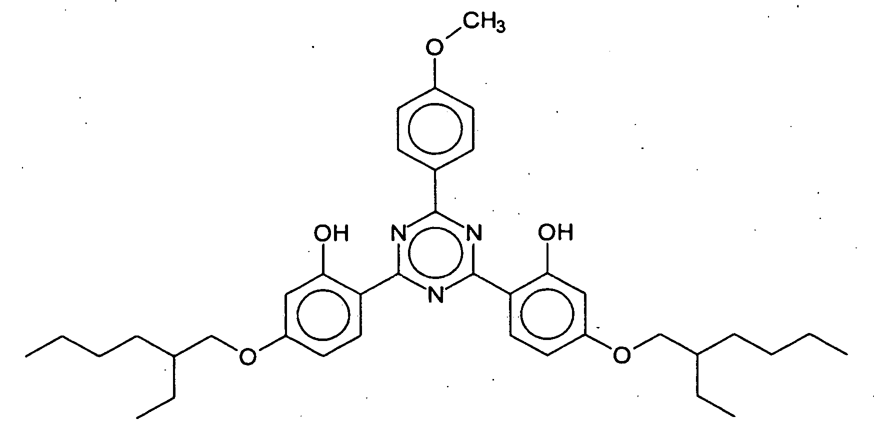 gekennzeichnet ist und unter der Handelsbezeichnung Tinosorb® S bei der CIBA-Chemikalien GmbH hergestellt und vermarktet wird.

Weitere vorteilhafte UV-A-Filtersubstanz im Sinne der vorliegenden Erfindung ist 2-(4'-(Diethylamino)-2'-hydoxybenzoyl)-benzoesäurehexylester, der unter der Bezeichnung "Uvinul® A+" von der Gesellschaft BASF AG hergestellt und vermarktet wird.

Weitere vorteilhafte UV-A-Filtersubstanzen im Sinne der vorliegenden Erfindung sind Benzoxazol-Derivate. Besonders bevorzugt ist das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0.

Vorteilhafte weitere UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, dass die mit dem Metall reagierende Verbindung zwei oder mehr Atomgruppierungen enthält, die als Elektronendonatoren wirken.

Den Nachteilen des Standes der Technik galt es also abzuhelfen.

Überraschenderweise zeichnen sich kosmetische Zubereitungen mit einem Gehalt von
i) 0,001 - 0,5 Gew.-% an Folsäure und/oder deren Derivaten, gewählt aus der Gruppe Dihydrofolsäure sind Tetrahydrofolsäure
ii) einem wirksamen Gehalt an 2,4-Bis-{[4-(2-ethyl-hexyloxy)-2-hydroxy]-phenyl}-6-(4-methoxyphenyl)-1,3,5-triazin (Tinosor® S),
iii) 0,01 - 0,4 Gew.-% an Ethylendiamintetraacetat
iv) bei einem pH > 6
durch erhöhte Stabilität der Folsäure aus und überkommen erfindungsgemäß die Nachteile des Standes der Technik

Die kosmetischen oder dermatologischen Zubereitungen können erfindungsgemäß wie üblich zusammengesetzt sein und zur Behandlung, der Pflege und der Reinigung der Haut und/oder der Haare und als Schminkprodukt in der dekorativen Kosmetik dienen.

Sie enthalten bevorzugt 0.001 Gew.-% bis 0.5 Gew.-%, besonders bevorzugt 0.005 Gew.-% bis 0.2 Gew.-%, insbesondere 0.01 bis 0.1 Gew.-%, bezogen auf das Gesamtgewicht der Zubereitungen, an Folsäure und/oder deren Derivaten.

Sie enthalten ferner bevorzugt 0.1 Gew.-% bis 3.5 Gew.-%, besonders bevorzugt 0.5 Gew.-% bis 3 Gew.-%, insbesondere 1- 2 Gew.-%, bezogen auf das Gesamtgewicht der Zubereitungen, an UVA-Filtern aus der Gruppe der Triazine, insbesondere 2,4-Bis-{[4-(2-ethyl-hexyloxy)-2-hydroxy]-phenyl}-6-(4-methoxyphenyl)-1,3,5-triazin (Tinosorb® S) oder 2,4-bis-[5-1(dimethylpropyl)benzoxazol-2-yl-(4-phenyl)-imino]-6-(2-ethylhexyl)-imino-1,3,5-triazin (CAS Nr. 288254-16-0).

Sie enthalten ferner bevorzugt 0.01 Gew.-% bis 0.4 Gew.-%, besonders bevorzugt 0.1 Gew.-% bis 0.4 Gew.-%, insbesondere 0.2 bis 0.3 Gew.-%, bezogen auf das Gesamtgewicht der Zubereitungen, an Ethylendiamintetraacetat.

Es ist erfindungsgemäß vorteilhaft, wenn das das molare Verhältnis aus (i) : (ii) : (iii) wie a : b : c gewählt wird, wobei a, b und c unabhängig voneinander rationale Zahlen von 1 bis 50 darstellen können. Bevorzugt wird ein Verhältnis von a (<1): b (6-40): c(1-8) gewählt.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindugsgemäß vorteilhaft, Folsäure und/oder deren Derivate in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, Folsäure und/oder deren Derivate in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Füllstoffe, Elektrolyte, organische Lösemittel oder Silikonderivate.

Die erfindungsgemäßen kosmetischen und dermatologischen Zubereitungen können vorteilhaft weitere Komplexbildner enthalten. Der oder die weiteren Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen, Phytinsäure, Natriumhexamethaphosphat, Ethylendiamindisuccinat, Nitrilotriessigsäure (NTA) und deren Anionen, Polyphenole aus der Gruppe der Flavonoide.

Der oder die weiteren Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bomylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-'(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- Dioctylbutylamidotriazon (INCI: Dioctylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoesäure-tris(2-ethylhexylester), auch: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride oder pflanzliche Öle, z.B. Caprylsäure/ Caprinsäuretriglyceride, Triisostearin, Avocadoöl, Kokosfettsäureglyceride, Macadamiaöl, Mandelöl, Jojobaöl, Rizinulsöl , Nachtkerzenöl oder Traubenkernöl
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren; z.B. Mineralöl, Dibutyladipat, Pentaerythrityltetraisostearat, 1-sopropylmyristat, Isopropylpalmitat, Isopropylstearat, Dicaprylylcarbonat, Caprylylether, Alkylbenzoat, Cetearylisononanoat, Alkyltartrat, Alkyllaktat, Polydecen, Isohexadecan, Octyldeceylmyristat, Squalan, Octylcocoat, Octyldodecanol, Butylenglykolcaprylat/caprat, Dicaprylylcarbonat
- Silikonöle, wie z.B. Cyclomethicon, Dimethicon, Phenyltrimeticon
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorteilhafte Verdickungsmittel für derartige Zubereitungen sind beispielsweise Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die Pemulen® Typen TR 1, TR 2 und TRZ von der Fa. Goodrich (Noveon).

Auch Carbopole sind vorteilhafte Gelbildner für derartige Zubereitungen. Carbopole sind Polymere der Acrylsäure, insbesondere auch Acrylat-Alkylacrylat-Copolymere. Vorteilhafte Carbopole sind beispielsweise die Typen 980, 981, 984 1342, 1382, 2984 und 5984. ebenso die ETD-Typen 2001, 2020, 2050 und Carbopol Ultrez 10, PVM/MA Decadien Crosspolymer (Handelsname Stabileze 06), Polyglycerylmethacrylat sowie Polyacrylamid.

Das Verdickungsmittel ist in dem Gel, der Dispersion bzw. der Emulsion z.B. in einer Menge zwischen 0,01 und 5 Gew.-%, bevorzugt zwischen 0,1 und 2 Gew.-%, enthalten.

Erfindungsgemäße als Emulsionen vorliegende Zubereitungen enthalten einen oder mehrere Emulgatoren. Diese Emulgatoren können vorteilhaft ausgewählt werden aus der Gruppe der nichtionischen, anionischen, kationischen oder amphoteren Emulgatoren. Beispiele sind u.a. PEG-40-Stearat , Sorbitanstearat, Glycerylstearatcitrat, Stearinsäure, oder Zuckerester, wie Polyglyceryl-3 Methylglucosedistearat:, Cetearylglucoside.

Weiterhin können zur Verbesserung des Hautgefühls Füllstoffe, wie z.B. Silica, Lauroyllysin, Polyethylen, Mica, Nylon oder Stärkederivate, wie z.B. Tapiokastärke, Aluminium Starch Octenylsuccinate, Distarch-phosphat eingesetzt werden.

Erfindungsgemäß können weitere Antioxidantien und Wirkstoffe, wie z.B. Vitamin A und Derivate (Vitamin-A-Palmitat), Vitamin C und Derivate (Ascorbylalmitat, Mg-Ascorbylphosphat), Vitamin E und Derivate, ungesättigte Fettsäuren und Derivate (Linolsäure, Linolensäure, Ölsäure), alpha-Glucosylrutin, Ubiquinon (Q10), Harnstoff, Pflanzenextrakte, Ginko, Soja, Aloe Vera, Süßholz, Hamamelis, Bisabolol, Ginseng, Gurke, Biotin, Carotinoide, Taurin, Isoflavonoide, Licochalkon, Dydydroxyaceton, Aminosäuren, Zinkoxid, Panthenol, Dioic acid, enthalten sein.

Weiterhin können zur Verbesserung der Hautpflegeeigenschaften Moisturizer wie z.B. Glycerin, Butylenglykol, Propylen Glykol, Methylpropandiol, Ethylhexylglycerin, Sorbitol oder Harnstoff zugesetzt werden.

Zuberereitungen gemäß der vorliegenden Erfindung können auch Konservierungsmittel und Konservierungshelfer, wie z.B. Phenoxyethanol, Parabene, Benzylalkohol, Hexamidin Diisethionat, Jodopropyl Butylcarbamat, Diazolidinyl Urea, Hydantoin, Sorbinsäure und ihre Salze, Benzoesäure oder Methyldibromoglutaronitril enthalten.

Es folgen vorteilhafte Ausführungsbeispiele der vorliegenden Erfindung.

### Tagescreme

| **Beispielrezeptur 1** | **Gew.-%** |
|---|---|
| Glycerylstearat | 2,5 |
| Stearylalkohol | 2 |
| PEG-40-Stearat | 1 |
| Cetylalkohol | 2 |
| Sheabutter | 2 |
| Füllstoffe (SiO₂, Nylon) | 1 |
| Tapiokastärke | 1 |
| 2,4-Bis-{[4-(2-ethyl-hexyloxy)-2-hydroxy]-phenyl}-6-(4-methoxyphenyl)-1,3,5-triazin (Tinosorb® S) | 2 |
| Ethlyhexylmethoxycinnamat | 2 |
| Phenoxyethanol | 0,4 |
| C₁₂₋₁₅ Alkylbenzoat | 3 |
| p-Hydroxybenzoesäurealkylester (Paraben) | 0,4 |
| Aristoflex AVC 0,2 (Ammonium Polyacryldimethyltaurid/Vinylformamid-copolymer) | |
| Kreatin | 0,4 |
| Folsäure | 0,05 |
| Glycerin | 8 |
| EDTA | 0,2 |
| Parfüm | 0,3 |
| Natronlauge (pH 7 eingestellt) | q.s. |
| Wasser 100% | ad 100 |

### DNA Schutzcreme (Tag)

| **Beispielrezeptur 2** | **Gew.-%** |
|---|---|
| Glycerylstearat | 3 |
| Stearylalkohol | 2 |
| PEG-40-Stearat | 1 |
| Sheabutter | 2 |
| 2,4-Bis-{[4-(2-ethyl-hexyloxy)-2-hydroxy]-phenyl}-6-(4-methoxyphenyl)-1,3,5-triazin (Tinosorb® S) | 2 |
| Ethlyhexylmethoxycinnamat | 5 |
| Phenoxyethanol | 0,4 |
| Dimethicon | 2 |
| Cyclomethicon | 4 |
| Titandioxid | 0,5 |
| Puderrohstoffe (Tapiocastärke, Nylon) | 3 |
| Tocopherylacetat | 1 |
| Retinylpalmitat | 0,3 |
| Parabene | 0,2 |
| Carrageenan | 0,1 |
| Polyacrylsäure | 0,1 |
| Kreatin | 0,2 |
| Folsäure | 0,05 |
| Glycerin | 8 |
| EDTA | 0,2 |
| Parfüm | 0,3 |
| Natronlauge (pH 6.5 eingestellt) | q.s. |
| Wasser | ad 100 |

### O/W-Emulsion

| **Beispielrezeptur 3** | **Gew.-%** |
|---|---|
| Glycerylstearatcitrat | 2 |
| Cetylalkohol | 3 |
| Sheabutter | 3 |
| Butylhydroxytoluol | 0,05 |
| 2,4-Bis-{[4-(2-ethyl-hexyloxy)-2-hydroxy]-phenyl}-6-(4-methoxyphenyl)-1,3,5-triazin (Tinosorb® S) | 2 |
| Ethylhexyltriazon | 2 |
| Squalan | 2 |
| Cyclomethicon | 5 |
| Dimethicon | 2 |
| Lauroyllysin | 2 |
| Carragenan | 0,15 |
| Carbomer | 0,1 |
| Kreatin | 0,2 |
| Folsäure | 0,03 |
| Glycerin | 8 |
| EDTA | 0,2 |
| Parfüm | 0,3 |
| Natronlauge (pH 6.5 eingestellt) | q.s. |
| Wasser | ad 100 |

### Pflegecreme

| **Beispielrezeptur 4** | **Gew.-%** |
|---|---|
| Hydrierte Kokosfettglyceride | 2 |
| Stearylalkohol | 2 |
| Cetylalkohol | 2 |
| Cetearylglucosid | 2 |
| Ethlyhexylmethoxycinnamat | 3 |
| 2,4-Bis-{[4-(2-ethyl-hexyloxy)-2-hydroxy]-phenyl}-6-(4-methoxyphenyl)-1,3,5-triazin (Tinosorb® S) | 2 |
| Butylhydroxytoluol | 0,04 |
| Myristylmyristat | 1 |
| Macadamiaöl | 0,5 |
| Sheabutter | 2 |
| Cyclomethicon | 2 |
| Polyacrylsäure | 0,1 |
| Kreatin | 0,4 |
| Folsäure | 0,04 |
| Glycerin | 9 |
| EDTA | 0,1 |
| Parfum | 0,3 |
| Tocopherylacetat | 0,5 |
| Ubiquinon (Q10) | 0,03 |
| Methylpropandiol | 2 |
| Natronlauge (pH 6.8) | q.s. |
| Wasser | ad 100 |

### Sonnenschutzcreme

| **Beispielrezeptur 5** | **Gew.-%** |
|---|---|
| Glycerylstearat | 2,5 |
| PEG-40-Stearat | 1 |
| Cetylalkohol | 2,5 |
| C₁₂₋₁₅ Alkylbenzoat | 2 |
| Hydrierte Kokosfettglyceride (Hydrogenated Coco Glycerides) | 2 |
| Octyldodecanol | 2 |
| Cyclomethicon | 2 |
| Bienenwachs | 1 |
| Ethylhexylmethoxycinnamat | 6 |
| Phenylbenzimidazolsulfonsäure | 2 |
| Natriumascorbylphosphat | 0,1 |
| Methylpropandiol | 2 |
| Diazolidinylharnstoff | 0,1 |
| Carrageenan | 0,1 |
| Carbomer | 0,1 |
| Glycerin | 7 |
| Folsäure | 0,03 |
| EDTA | 0,2 |
| 2,4-Bis-{[4-(2-ethyl-hexyloxy)-2-hydroxy]-phenyl}-6-(4-methoxyphenyl)-1,3,5-triazin (Tinosorb® S) | 2 |
| Natronlauge (pH 6.8) | q.s. |
| Wasser ad | 100 |

## Patentansprüche

1. Kosmetische oder dermatologische Zubereitungen mit einem Gehalt von
i) 0,001 - 0,5 Gew.-% an Folsäure und/oder deren Derivaten, gewähltaus der Gruppe Dihydrofolsäure und Tetrahydrofolsäure
ii) einem wirksamen Gehalt an
2,4-Bis-{[4-(2-ethyl-hexyloxy)-2-hydroxy]-phenyl}-6-(4-methoxyphenyl)-1,3,5-triazin,
iii) 0,01 - 0,4 Gew.-% an Ethylendiamintetraacetat
iv) bei einem pH > 6.

2. Kosmetische Zubereitungen nach Anspruch 1, **dadurch gekennzeichnet, dass** sie zusätzlich 0,001 Gew.-% bis 1 Gew.-% Kreatin enthalten.

3. Kosmetische oder dermatologische Zubereitungen, nach Anspruch 1, **dadurch gekennzeichnet, dass** sie 0,001 Gew.-% bis 0,5 Gew.-%, besonders bevorzugt bevorzugt 0,005 Gew.-% bis 0,2 Gew.-%, insbesondere 0,01 bis 0,1 Gew.-%, bezogen auf das Gesamtgewicht der Zubereitungen, an Folsäure und/oder deren Derivaten enthalten.

4. Kosmetische oder dermatologische Zubereitungen, nach Anspruch 1 oder 2, **dadurch gekennzeichnet, dass** sie 0,1 Gew.-% bis 3,5 Gew.-%, besonders bevorzugt 0,5 Gew.-% bis 3,0 Gew.-%, insbesondere 1,0 bis 2,0 Gew.-%, bezogen auf das Gesamtgewicht der Zubereitungen, an 2,4-Bis-{[4-(2-ethyl-hexyloxy)-2-hydroxy]-phenyl}-6-(4-methoxyphenyl)-1,3,5-triazin, enthalten.

5. Kosmetische oder dermatologische Zubereitungen, nach einem der vorstehenden Ansprüche, **dadurch gekennzeichnet, dass** sie bevorzugt 0,01 Gew.-% bis 0,4 Gew.-%, besonders bevorzugt 0,1 Gew.-% bis 0,4 Gew.-%, insbesondere 0,2 bis 0,3 Gew.-%, bezogen auf das Gesamtgewicht der Zubereitungen, an Ethylendiamintetraacetat enthalten.

6. Kosmetische oder dermatologische Zubereitungen, nach einem der vorstehenden Ansprüche, in denen das molare Verhältnis aus (i) : (ii) : (iii) wie a : b : c gewählt wird, wobei a, b und c unabhängig voneinander rationale Zahlen von 1 bis 50 darstellen können.

## Claims

1. Cosmetic or dermatological preparations with a content of
i) 0.001-0.5% by weight of folic acid and/or derivatives thereof, selected from the group consisting of dihydrofolic acid and tetrahydrofolic acid,
ii) an effective content of 2,4-bis{[4-(2-ethyl-hexyloxy)-2-hydroxy]phenyl}-6-(4-methoxyphenyl)-1,3,5-triazine,
iii) 0.01-0.4% by weight of ethylenediamine tetraacetate
iv) at a pH > 6.

2. Cosmetic preparations according to Claim 1, **characterized in that** they additionally comprise 0.001% by weight to 1% by weight of creatine.

3. Cosmetic or dermatological preparations according to Claim 1, **characterized in that** they comprise 0.001% by weight to 0.5% by weight, particularly preferably 0.005% by weight to 0.2% by weight, in particular 0.01 to 0.1% by weight, based on the total weight of the preparations, of folic acid and/or derivatives thereof.

4. Cosmetic or dermatological preparations according to Claim 1 or 2, **characterized in that** they comprise 0.1% by weight to 3.5% by weight, particularly preferably 0.5% by weight to 3.0% by weight, in particular 1.0 to 2.0% by weight, based on the total weight of the preparations, of 2,4-bis{[4-(2-ethylhexyloxy)-2-hydroxy]phenyl}-6-(4-methoxyphenyl)-1,3,5-triazine.

5. Cosmetic or dermatological preparations according to one of the preceding claims, **characterized in that** they comprise preferably 0.01% by weight to 0.4% by weight, particularly preferably 0.1% by weight to 0.4% by weight, in particular 0.2 to 0.3% by weight, based on the total weight of the preparations, of ethylenediamine tetraacetate.

6. Cosmetic or dermatological preparations according to one of the preceding claims, in which the molar ratio of (i) : (ii) : (iii) is selected as a : b : c, where a, b and c, independently of one another, may be rational numbers from 1 to 50.

## Revendications

1. Compositions cosmétiques ou dermatologiques, présentant une teneur de
i) 0,001-0,5% en poids d'acide folique et/ou ses dérivés, choisis dans le groupe de l'acide dihydrofolique et de l'acide tétrahydrofolique
ii) une teneur active en 2,4-bis-{[4-(2-éthylhexyloxy)-2-hydroxy]-phényl}-6-(4-méthoxyphényl)-1,3,5-triazine,
iii) 0,01-0,4% en poids d'éthylènediaminetétraacétate
iv) à un pH > 6.

2. Compositions cosmétiques selon la revendication 1, **caractérisées en ce qu'**elles contiennent en outre 0,001% en poids à 1% en poids de créatine.

3. Compositions cosmétiques ou dermatologiques selon la revendication 1, **caractérisées en ce qu'**elles contiennent 0,001% en poids à 0,5% en poids, de manière particulièrement préférée 0,005% en poids à 0,2% en poids, en particulier 0,01 à 0,1% en poids, par rapport au poids total des compositions, d'acide folique et/ou ses dérivés.

4. Compositions cosmétiques ou dermatologiques selon la revendication 1 ou 2, **caractérisées en ce qu'**elles contiennent 0,1% en poids à 3,5% en poids, de manière particulièrement préférée 0,5% en poids à 3,0% en poids, en particulier 1,0 à 2,0% en poids, par rapport au poids total des compositions, de 2,9-bis{[4-(2-éthylhexyloxy)-2-hydroxy]-phényl}-6-(4-méthoxyphényl)-1,3,5-triazine.

5. Compositions cosmétiques ou dermatologiques selon l'une quelconque des revendications précédentes, **caractérisées en ce qu'**elles contiennent de préférence 0,01% en poids à 0,4% en poids, de manière particulièrement préférée 0,1% en poids à 0,4% en poids, en particulier 0,2 à 0,3% en poids, par rapport au poids total des compositions, d'éthylénediaminetétraacétate.

6. Compositions cosmétiques ou dermatologiques selon l'une quelconque des revendications précédentes, dans lesquelles le rapport molaire (i) : (il) : (iii) est choisi comme a:b:c, où a, b et c peuvent représenter indépendamment l'un de l'autre des nombres rationnels de 1 à 50.
